# EUROPEAN PATENT SPECIFICATION

(11) **EP 3 808 245 B1**
(45) Date of publication and mention of the grant of the patent: **17.05.2023**
(21) Application number: 19204168.9
(22) Date of filing: 18.10.2019
(51) Int. Cl.: A61B 1/00, A61B 1/018, A61B 1/05

(54) **ENDOSCOPE**
ENDOSKOP
ENDOSCOPE

(43) Date of publication of application: 21.04.2021
(73) Proprietor: EndoScope sp. z o.o., 87-100 Torun (PL)
(72) Inventor: Mankowski, Patrycjusz, 03-289 Warszawa (PL); Mankowski, Szymon, 87-100 Torun (PL)

(56) References cited:
- WO-A1-2018/084730
- US-A1- 2018 146 839

## Description

The subject of this invention is a flexible endoscope with an independent optical-illumination module applicable for conducting specialized tests and diagnostic and therapeutic procedures through natural body openings, in which the optical-illumination part is an independent element of the device.

Rigid endoscopes and flexible endoscopes, including bronchoscopes, gastroscopes and colonoscopes, are known. The flexible endoscopes are equipped with a handle, which through a guide joint, usually in the form of a conical culvert, is connected to a insertion tube in the form of a flexible cable with a bundle of functional channels, which is terminated with a distal tip. The movement of the insertion tube is ensured by tension cables drawn along the entire length of the endoscope and controlled by knobs located in the handle. As functional channels, a biopsy-suction channel, an air channel, a channel for flushing optical elements, and an observation field flushing channel powered from a separate pump are commonly used. Optical fibers and electrical wires are also routed through the endoscope to provide optical and lighting functions, including camera operation. In known endoscopes, function channel bundles, optical fiber bundles and electrical bundles derived from the handle and are connected to the peripheral devices through a bundle of connectors, thus ensuring transmission through appropriate media of all functions performed throughout the entire length of the endoscope up to the tip of the endoscope.

Flexible endoscopes are known, in which the flexible insertion tube together with the tip, the culverts, handles, connection bundles constitute integral device where none of part can be separated. In the most commonly used devices, the technical elements performing the optical-lighting function are not fully separated from the functional channels. Due to the fact that the biopsy-suction channel is often damaged, it also leads to serious damage to the optical-lighting parts.

Endoscopic examinations have great influence in diagnostics, but they can be the cause of serious infections caused by inattention during cleaning, disinfection, sterilization due to the problematic construction of the device. Moreover, due to the high cost of endoscopes, these devices are most often used as reusable devices. After the common examination, the endoscope is thoroughly cleaned, high-level disinfected, sterilized. This requires both adequate resources, equipment and specialized staff. In terms of contamination and infection the most dangerous elements are long functional channels, which due to their dimensions and the materials require specialized disinfection and sterilization methods. Statistical data indicates that almost every case of documented infection during endoscopic examination was caused by mistakes in cleaning, disinfection or sterilization. Many disinfectants that are successfully used for simple medical instruments do not work with endoscopes. Alcohols, aldehydes and high temperature most commonly used in disinfection can cause the fixation of impurities and, consequently, the formation of biofilms, especially on hard to reach surfaces such as narrow functional channels. These measures are often harmful to the health of personnel.

The growing epidemiological threat associated with hospital infections, necessitates more effective methods of preventing infection transmission. The most reliable of these is the use of disposable medical devices that are disposed of after medical treatment.

For this reason, endoscopic technology is still under development and there is a great need to find solutions that combine ergonomics, guarantee safe use, are inexpensive, guarantee sterility at a satisfactory level determined by currently functioning medical standards are still being sought. Single-use endoscopy can provide solutions to these problems.

Among the many solutions in the design of endoscopes, there cannot be found one that guarantees the sterility of the device before reuse. In addition, every known endoscopy systems are expensive and also the proposed new solutions do not provide satisfactory reduction of the high costs of the medical procedures. Few of the proposed solutions concern ergonomics, which directly affects the quality and duration of medical procedures. Single-use endoscopy seems to be the right direction and the technical ideas proposed in this document are the answer to all of the above problems. This application describes the optical-illumination module, which is an independent part of endoscopes and the organization of space inside the endoscope insertion tube. This module mainly contains an optical fibers bundle, electrical wires bundle, CCD or CMOS camera or matrix or other device with an analogous function, mechanical and biological protection in the form of a flexible module sheath, an electro-optical connector linking a CCD matrix, CMOS or micro-camera with appropriate external devices such as e.g. analog-to-digital converter, controller and computer equipment and with a light source also. The optical-illumination module is the most expensive part of the endoscope and is designated for multiple use, while the other parts of the endoscope, such as a handle with knobs, the insertion tube with functional channels and tension cables are disposable and can be made mostly of selected polymers such as polypropylene, polyethylene, silicones . The disposable parts of the endoscope together constitute the working module. The optical-illumination module due to its multiple use is completely hermetic, designed this way it does not have any channels, openings or pockets where organic materials could remain after medical treatment. Infections in classic endoscopes mainly result from inaccurate cleaning, because biological material may remain inside the functional channels, which, even after a high level of disinfection, may still be contagious. Classic endoscopes often unseal after many cycles of use, carrying the same risk to patients. In addition, unsealing in a classic endoscope means a failure that immediately excludes the use of this device until the end of the service procedure. Any break of the tension cable, what often happens in classic endoscopes, cracking the optical fibers, failure of the camera have same results. Prior art document WO 2018/084730 discloses an endoscope comprising a handle connected to a flexible insertion tube in the form of a flexible cable with a bundle of functional channels and tension cables for moving the tip of the insertion tube, said endoscope further comprising a removable and independent optical illumination module, wherein said insertion tube includes an air channel, a suction channel, a water channel and an optical illumination channel, and wherein said insertion tube includes a flexible part that leads from the handle, a bending part located distal to the flexible part and a distal tip, wherein said optical illumination module comprises a camera enclosed in a capsule, an optical fiber bundle and an electric wires bundle, wherein said bundles are guided in said optical illumination module within an optical illumination cable which extends from the proximal side of the capsule, wherein an electro-optical connector which is a connector for peripheral devices is located at the proximal-most end of said cable and terminates said cable and in which the optical fiber bundle and the electric wires bundle terminate, said optical illumination module being configured to be guided through the entire length of the insertion tube within the optical illumination channel, wherein along the length of the distal tip of the insertion tube the optical illumination channel has a diameter equal to or greater than the diameter of the capsule of the optical illumination module, wherein the optical illumination cable of the optical illumination module which extends from the proximal side of capsule is of a length such that when said cable is guided through the bending part and the flexible part of the insertion tube the electro-optical connector thereof is led out of an optical channel output located in the handle of the endoscope and which communicates with the optical illumination channel of the insertion tube such that said connector extends outside said output, wherein the optical illumination cable is smaller in diameter than the optical illumination channel but adapted to the size of the optical-illumination channel in the flexible part and the bending part.

Patent application EP 1765146 discloses a micro-camera embedded in a sealed housing that is reusable for other medical devices, including an endoscope. This element includes the housing, lenses, semiconductor sensor, electronic controllers, internal electrical connectors. The internal and external electrical connectors contain a number of pins or sockets arranged in a pattern-matching manner on opposite surfaces, so that the camera head can be connected and disconnected from the appropriate device. The micro-camera is used in a reusable manner, and the other components of the device in a single use. Optical components in the endoscope, such as micro-cameras, can be separated from other parts of the endoscope, including lighting means. Reusable visual elements are washed, disinfected or sterilized. According to the disclosed invention, the electrical connectors of the microcontroller may be exposed to body fluids and during disinfection and sterilization to hot steam or chemicals that may require additional protection for micro-camera for endoscopic applications. This solution is expensive and does not differ significantly in economic terms from the existing solutions on endoscopic market, where disposable endoscopes are designed as devices without any reusable components. Camera is the only element that can be saved here due to maintaining high sterility standards. According to the proposed solution disclosed in this application, significantly more components such as fiber optic bundle, electric wires and connectors can be reused.

From the patent specification WO1993015648 an endoscope with a flexible and detachable insertion tube from the handle is known. The insertion tube consists of a reusable optical element inserted from the proximal side to the disposable insertion tube equipped with function channels and tension cables and terminated with a transparent window. The handle remains as reusable part, which makes sterilization difficult, especially when the video monitor connected to the control module cannot be sterilized. The tension cables must be disconnected from the control module to replace the new disposable part, and when installing a new part, the tension cables must be reattached to the control module.

As above, US5483951 discloses a disposable multi-channel sheath slipped over the insertion tube of a reusable endoscope. The multi-channel cover is not fitted with tension cables.

From the description of the invention application 419328, a flexible endoscope is known, which is characterized by that it is equipped with a detachable and independent optical-illumination module, which includes a flexible cable terminated with a capsule, and optical fiber bundles and electric bundles terminated in the flexible cable peripheral devices, while the micro-camera with the lens system and the ends of the optical fiber bundle and the electric bundle are located in the capsule. An optical-illumination channel is formed inside the insertion tube, however, after installing the optical-illumination module in the endoscope, the capsule is stably located in the distal tip formed by movably connected parts. The above application solves the stable mounting of the optical-illumination module capsule in the distal end of the endoscope when inserting the optical-illumination module from the handle side. This forces the use of a distal tip composed of moving parts so that the capsule can be mounted in it. This makes it difficult to make the tip and creates the potential for splitting parts during the medical procedure. The insertion of the optical-illumination module capsule through the handle and the entire length of the insertion tube into the tip causes an increase in the diameter of the insertion tube and significantly hinders its use in endoscopes with small insertion tube diameters such as bronchoscopes. The above solution also does not provide alternative ways of conducting tension cables in the insertion tube.

The disposable endoscope with an independent optical-illumination module according to the proposed solution is much less susceptible to malfunction than a classic endoscope. The most susceptible elements of the endoscope, such as a insertion tube with channels, tension cables, a mechanism controlling the tension cables with a handle are disposable. There is also no risk of leakage of the sight glass insertion tube because it is a disposable element and the channels running in it do not connect with the optical-illumination module. The reusable optical-illumination module after cleaning and sterilization can be reused with a new sterile working module.

The optical-illumination module according to the invention is an independent unit, independent from the insertion tube and the handle but is an indispensable part of the flexible endoscope. After installing the optical-illumination module inside the insertion tube, the endoscope is ready to use. The installation of the optical-illumination module in the endoscope is easy and quick, which is an important aspect for medical personnel using disposable endoscopes. The installation of the optical-illumination module inside the disposable module is carried out by introducing optical-illumination module into the optical-illumination channel of the insertion tube through the distal tip, until the capsule of the module is mounted and locked out in the distal tip, and the electro-optical connector of the optical-illumination module will be led out from the insertion tube through the optical channel output located in the handle outside the endoscope. After the medical procedure, the optical-illumination module is removed from the endoscope by slight pushing the optical-illumination module from the channel, the locked capsule of the optical-illumination module along with the rest of the module is ejected enough to allow further removal whole module. Then, the removed optical-illumination module undergoes a cleaning and sterilization process. The advantage of such an endoscope approach is that the optical-illumination modules are simpler in design, they do not have gaps and channels, thus they do not require complicated and time-consuming manual cleaning procedures. Inaccurate washing and cleaning in the classic endoscopy results infections. The cost of manufacturing and using an optical-illumination module is lower than that of classic endoscopes and takes up much less space and does not require special storage conditions. In this way, with several optical-illumination modules, several medical procedures can be carried out without sterilizing between them and sterilizing the modules collectively for several modules together. Several optical-illumination modules can fit in a common autoclave or ultrasonic washer and sterilizing and cleaning more devices at the same time shortens the time to prepare for subsequent treatments and at the same time decreases the overall costs of medical procedures. The flexible endoscope according to the invention is much more reliable compared to classic endoscopes, and the compact design of reusable parts brings the advantages of easy cleaning and safe reuse.The design of the optical-illumination module according to the invention also allows for use of the same optical-illumination module with endoscopes of different lengths and diameters of the insertion tube, among others, such as gastroscopes, colonoscopes, bronchoscopes.

The endoscope according to the invention, due to the modular construction, also gives several possibilities in organizing the space inside the single-use insertion tube to save space and minimize its diameter. First, introducing optical-illumination module through the distal tip causes that the element with the largest dimensions, which is the capsule with a micro-camera, is not passed through the insertion tube. This creates opportunity for reducing the diameter of the optical-illumination channel and, consequently, the entire insertion tube. Secondly, the channels can be shared between the tension cables and the media. The following solutions are proposed: tension cables guided through the optical-illumination channel by using the clearance between the optical-illumination module and the inner wall of the channel, tension cables guided through the air channel, tension cables guided throuhg a dedicated and channel for tension cables. The air channel is suitable for guiding the tension cables, because the tension cables are not able to stop gaseous media. The air channel or optical-illumination channel does not have to be hermetic when using a disposable endoscope. The air channel, dedicated tension cable channel have a small diameter, so in order to translate the pulling force into the effective deflection of the distal tip of the insertion tube, the bending part of the insertion tube has four separate channels for each cable. These channels are routed close to the perimeter of the bending part of the insertion tube. Preferably, the angle between them is 90 degrees.. Such arrangement of tension cables in the flexible section enables the distal tip to be effectively bent in all required directions; right, left, up, down.

To separate and guide each tension cable into a single channel in the bending part, a tension cable splitter is applied which is placed between the bending part and the flexible part of the insertion tube. The air channel with routed tension cables or the dedicated tension cable duct have a continuation in the splitter, which finally has one proximal entry duct, and distally four exits for smaller diameter ducts for tension cables that run further along the perimeter of the bending part of the insertion tube and throughout its length to distal tip.

The invention, which is defined in the appended claims, is illustrated in detail in drawings of embodiments of the invention.
Fig. 1. Flexible endoscope withpulled out optical-illumination module.
Fig. 2.An example of an optical-illumination module with a stiffening rod having constant diameter.
Fig. 3.An example of an optical-illumination module with a stiffening rod which has a variable diameter.Fig. 4.An example of an optical-illumination module with a spiral stiffening rod.
Fig. 5. An embodiment of the capsule with a spring fold installed in the optical-illumination channel.
Fig. 6. Cross-section of the insertion tube, embodiment with separately guided tension cables, common approach.
Fig. 7. Cross-section of the insertion tube, an embodiment with tension cables guided through the optical-illumination channel.
Fig. 8. Cross-section of the insertion tube, embodiment with tension cables guided through the air channel.
Fig. 9. Cross-section of the insertion tube, embodiment with tension cables guided through the tension cables channel.
Fig. 10. Splitter and distribution view of the tension cables inside it.
Fig. 11. View inside the insertion tube with the optical-illumination module installed inside it (foldlocked in the locking groove).
Fig. 12. Minimum bending radius of the optical-illumination module.
Fig.13.Cross-section through the insertion tube with an optical-illumination module installed in the optical-illumination channel.

### Building and function - example 1.

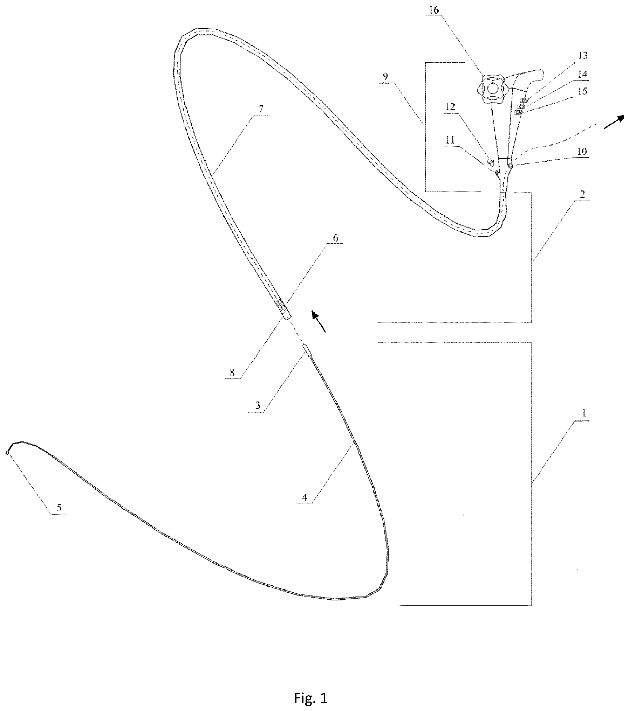

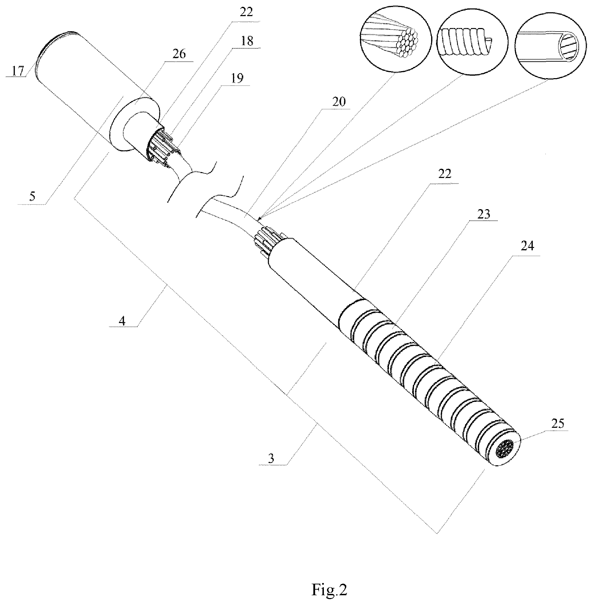

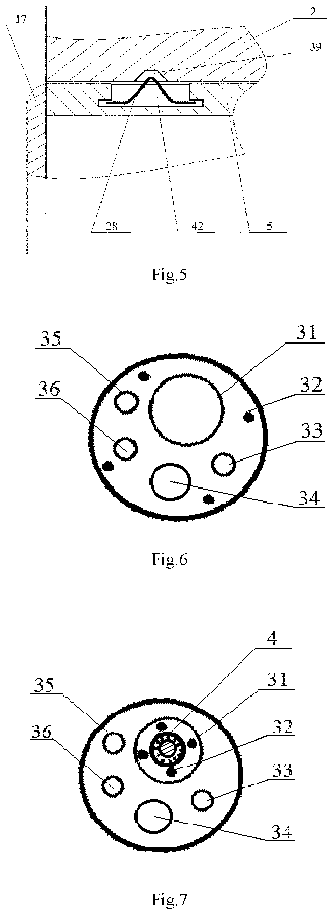

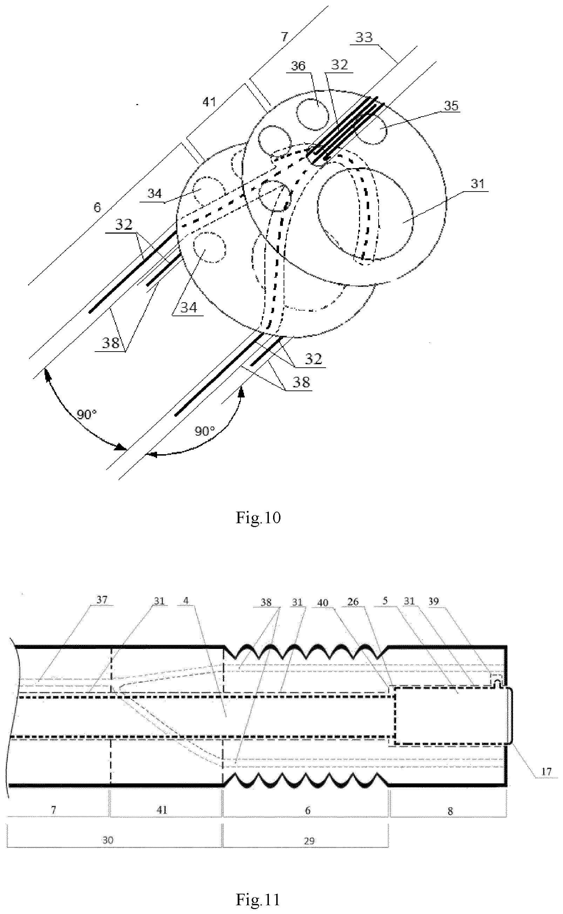

An endoscope according to the invention as shown in Fig. 1, consists of such elements as the insertion tube 2 intended to be inserted through the stomata, the handle 9 which includes a mechanism with knobs 16 responsible for controlling the distal tip 8 of the insertion tube 2 by pulling the appropriate tension cables 32, the optical-illumination module 1 responsible for transmitting the image from inside the patient's body and providing the light to the place of observation in the patient's body. Insertion tube 2 has the shape of a long pipe made of flexible materials, e.g. silicone, polyethylene of selected density, glass fiber reinforced latex to ensure its free bending and is equipped with tension cables 32 guided inside the insertion tube with separate channels for each tension cable (not shown in the drawing), as well as in functional channels such as; air channel 33, tool channel 24, suction channel 35, water channel 36, optical-illumination channel 31 - Fig. 6. The insertion tube has a flexible part 7 leading from the handle to the bending part 6, the bending part 6, which can be bent by pulling the tension cables and located between the flexible part 7 and the distal tip 8 and the distal tip 8 located at the end of the bending part 6. The optical-illumination channel 31, which is led through the entire length of the insertion tube 2, the optical-illumination module 1 is inserted starting from the side of the distal tip 8 passing successively through the bending part 6 of the insertion tube, the flexible part 7 of the insertion tube. Through the optical channel output 10 located in handle 9 an electro-optical connector 3 comes out- Fig. 1. To stop the capsule 5 of the optical-illumination module 1 moving further inside the insertion tube 2 and mount it in the distal tip 8 - Fig. 11, the optical-illumination channel 31 is narrower in bending part 6 and in flexible part 7 and wider at the distal tip 8. The created narrowing of the optical-illumination channel 31 between the bending part 6 of the insertion tube and the distal tip 8 creates a blocking flange 40. The optical-illumination channel 31 on the section of the distal tip 8 has a diameter equal or greater than the diameter of the capsule 5 of optical-illumination module 1, and on the section of flexible part 7 and bending part 6 has a diameter smaller than the diameter of the capsule 5. Because the capsule 5 is an element of the optical-illumination module 1 having the largest diameter, during introducing the optical-illumination module 1 into optical-illumination channel 31 must occur a moment when the rear wall of the capsule 26 Fig 2 touches the blocking flange 40 of the optical-illumination channel, which prevents further movement of the optical-illumination module along the channel Fig. 11.

The optical-illumination module 1 is designed as a stand-alone and removable unit, adapted for mounting in endoscopes of various lengths and diameters. The optical-illumination module 1, according to the first embodiment, consists of three main parts: capsules 5 , optical-illumination cable 4, electro-optical connector 3, as shown in Fig. 1. Capsule 5 forms a housing for optical elements and preferably have cylindrical shape, equipped on the distal side with a lens 17 and the tips of fiber optic bundles 19 (not shown in the drawing). Inside the capsule 5, the micro-camera with the electrical wires bundle 18 and one end of the fiber bundle 19 are hermetically sealed. The electrical wires bundle 18 and optical fibers bundle 19 are guided through the capsule back wall 26 to the optical-illumination cable 4 and terminated in the electro-optical connector 3 which can be plugged into peripheral devices such as a light source and a dedicated video driver or computer. The stiffening rod 20 is also attached to the capsule back wall 26 as shown in Fig 2.

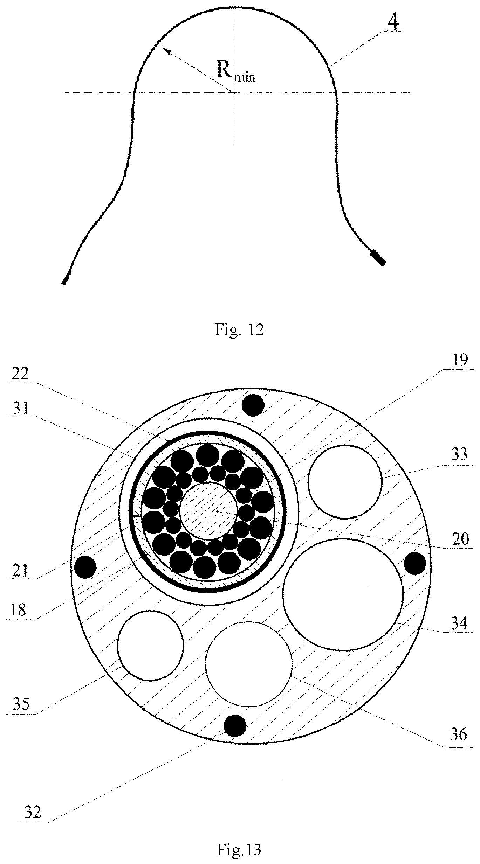

The optical-illumination cable 4 has a smaller diameter than the each segment of the optical-illumination channel 31 and consists of a stiffening rod 20 guided along the entire length of cable, preferably in a center of the cable, a bundle of optical fibers 19 surrounding the rod 20, an electrical wires bundle 18 surrounding the bundle of optical fibers 19 together with the rod 20, elastic sheath 22, which is the outer layer of the cable 4, as shown in Fig. 13 An elastic sheath 22 made of suitably elastic material such as latex, silicone or other polymer complying with medical standards with selected physical parameters which do not limit a bending the cable and at the same time protects the interior of the module against contaminated body fluids and gases. The stiffening rod 20 is in the form of a preferably elastic cord or elastic spiral, or a flexible rod preferably made of metal, e.g. stainless steel or polymer or composite with selected elasticity, e.g. polyamide or a mixture of acrylonitrile, butadiene and styrene (ABS) with carbon fiber reinforcement as shown in Fig2.

The stiffening rod 20 according to this embodiment has the same diameter over its entire length. The diameter of the stiffening rod 20 and the material from which it is made determine the degree of flexibility of the entire optical-illumination cable 4, which affects the stiffness of the entire insertion tube 2. The stiffening rod 20 helps to install the optical-illumination module 1 inside the insertion tube 2 when it is inserted into the optical-illumination channel 31. A suitably stiff optical-illumination cable 4 facilitates assembly the module into the insertion tube because it does not bend or twist in a certain distance during pulling into the optical-illumination channel 31. The stiffening rod 20 is permanently connected, e.g. by welding, soldering, gluing on the distal end to the capsule 5 and with the electro-optical connector 3 at the proximal end, and play a structural role as a strengthening element of the optical-illumination module, also preventing the module from breaking against stretching force. The optical-illumination cable 4 at the distal end is connected to the capsule 5, and the proximal end to the electro-optical connector 3.

The electro-optical connector 3 has a cylindrical shape smaller than each section of the optical-illumination channel 31. The side wall of the electro-optical connector contains electrical conductors 23 made of metal in the shape of rings and electrical insulators 24 made of non-conductive material also with ring shape. The electrical conductors 23 are adjusted to the contacts in the socket from the peripheral devices providing power connection for micro-camera and transferring image signal (not shown in the drawings). Inside the electro-optical connector, electrical conductors 23 are connected to the ends of the electrical wires bundle 18. The rear wall of the connector 3 is equipped with a transparent window 25 fixed in it hermetically. The ends of the optical fibers bundle 19 are led to the transparent window 25.

After connecting the electro-optical connector 3 to the light source, the transparent window allows the light for passing through the fiber optic bundles to the examined body cavity. A stiffening rod 20 and the elastic sheath 22 are attached to the front wall of the electro-optical connector. The electrical wires bundle 18 and the optical fibers bundle 19 pass through the front wall of the electro-optical connector. The electro-optical connector is hermetically sealed and the hermeticity between the connector and the electro-optical cable is ensured by anelastic sheath 22 as shown in Fig. 2.

Mounting and removing the optical-illumination module into and from the endoscope.

To install the optical-illumination module inside the insertion tube 2 what is shown in Fig. 1, the module should be inserted through the distal tip 8 into the optical-illumination channel 31. The order of installing the optical-illumination module in the endoscope is as follows: the electro-optical connector 3 is directed to the distal tip 8 of the insertion tube 2, then the module is inserted into the optical-illumination channel 31 of the insertion tube2 until the electro-optical connector 3 came out from the optical-illumination channel 31 through the output of the optical channel output 10. Then, grasping the electro-optical connector 3 or this section of the electrical-optical cable 4 which with the electro-optical connector 3 came outside the channel through the optical channel output 10, the optical-illumination module 1 can be pulled into the optical-illumination channel 31 to its final position. Installation continues until part of the capsule back wall 26 rests on the blocking flange 40 inside the optical-illumination channel, preventing from further movement of the module along the channel as shown in Fig. 11. The stability of the capsule 5 in the distal tip 8 is guaranteed bya well-fitting shapes and close dimensions of the capsule 5 and the optical-illumination channel 31 in the distal tip 8. After installing the optical-illumination module inside insertion tube 2, the endoscope is ready to use.

A removal phase of the optical-illumination module 1 from the endoscope is in reverse order to mounting phase. By pushing the electro-optical cable 4 at the side of the optical channel output 10 into the optical-illumination channel, the capsule 5 is slide out from the distal end 8. This maneuver facilitates the stiffening rod 20 inside the optical-illumination cable. Then, by grasping the capsule 5, the optical-illumination module 1 is removed from the endoscope by pulling it out from the optical-illuminationchannel 31 insertion tube 2.

### Embodiment and function - example 2.

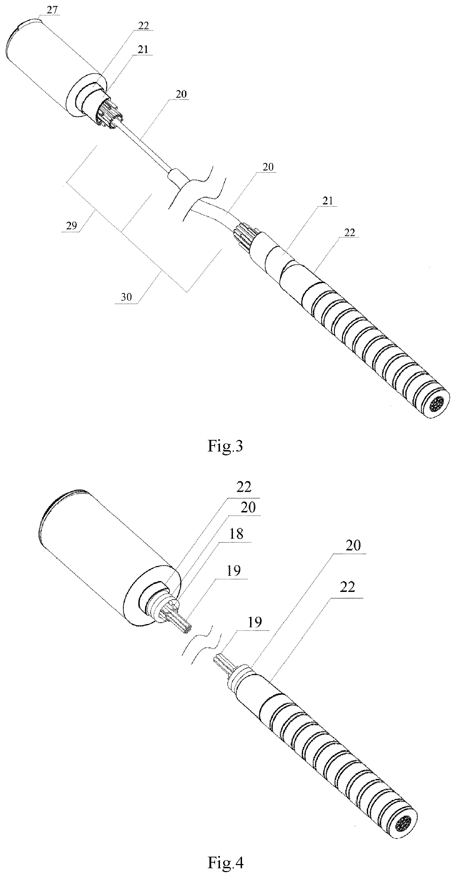

In this example, the function and design of the optical-illumination module 1 and the insertion tube is analogical to example 1 with the following changes:
The optical-illumination cable 4 has a smaller diameter than each section of the optical-illumination channel and consists of a stiffening rod 20,optical fibers bundle 19, electrical wires bundle 18, spiral protective band 21, hermetic elastic sheath 22, which is the outer layer of the cable as shown in Fig .3 Spiral protective band 21 is attached to the capsule back wall 26 with one end and attached to the front wall of the electro-optical connector 3 with the other end. The spiral protective band 21 is made of metal such as stainless steel or a polymer like polyamide and it has the form of a thin narrow band twisted into a spiral shape and in the space created inside the spiral a stiffening rod 20, optical bundle 19, electrical bundle 18 is passed through. The spiral protective band 21 prevents from mechanical damage to the optical and electrical bundles during installation or uninstallation the optical-illumination module 1. It also sets the minimum bend radius of the electro-optical cable 4 as shown in Fig. 12, which is important for instance during sterilization procedure when the entire module must be rolled up to fit into the autoclave chamber. This radius determines the maximum bending capability of the optical-illumination module 1 without the risk of breaking optical fibers. The elastic sheath 22 provides hermeticity of the optical-illumination cable 4 and is partly overlapping the side wall of the capsule 5 and the side wall of the optical-optical connector 3 (not shown on figs.). The optical-illumination cable 4 according to the present embodiment has a separate bending section 29 and a flexible section 30 as shown in Fig. 3. Both sections have a stiffening rod 20 of a different diameter. This segment of the stiffening rod 20 which corresponds to the bending section 29 has a smaller diameter than the segment of the stiffening rod 20 corresponding the flexible section 30, and the length of the bending section and flexible section of the module 1 corresponds to the lengths of the bending part 6 of the insertion tube and the flexible part 7 of the insertion tube what is shown in Fig. 1 and Fig. 11 This modification provides variable stiffness of the module 1, the purpose of which is to reduce the stiffness of the optical-illumination module over the flexible section of module 29 and, consequently, to reduce the stiffness of the bending part 6 of the insertion tube (2). This translates into less force required to manipulate the distal tip 8 of the insertion tube 2. Additional improvement of this property can be achieved by using various materials, for example by making the banding section of module 29 from a more flexible material than the flexible section of module 30.

In this embodiment, the capsule 5 equipped with a fold 27 matched in size and shape to a locking groove 39 located in the optical-illumination channel of the distal tip 8 what is shown in Fig. 11.

The fold 27 has the shape of a thin fold over a small section of the capsule edge. Locking the fold 27 into the locking groove 39 improves the stability of the attachment of the capsule 5 in the distal tip 8 - Fig. 11. Positioning the fold 27 into the locking groove 39 and retraction it is possible because of the elasticity and of the material from which the insertion tube is made of, e.g. silicone, PVC and also by a small height of the fold.

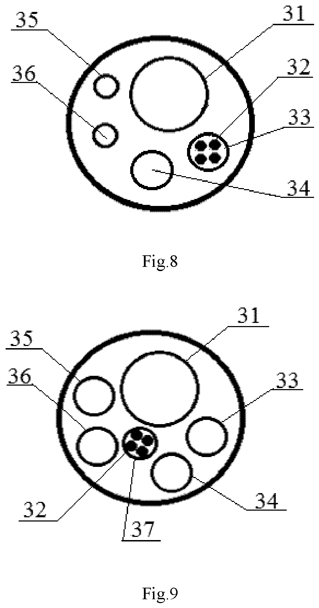

In this embodiment of the insertion tube, the tension cables are routed through the air channel 33 starting from the handle 9 to the distal tip 8 as shown in Fig. 8.

Because the air channel is narrow, an additional splitter 41 is used. The splitter is part of the insertion tube made of metal, selected polymer or flexible material such as silicone or rubber. The splitter is designed to provide the continuation of the insertion tube's functional channels (optical -illumination channel 31,air channel 33,tool channel 34,suction channel 35, water channel 36 ) and to separate a single channel where the tension cables are guided, into four separate tension cable channels of bending part 38 located close to the perimeter of the bending part 6 of the insertion tube 2 as shown in Fig. 10. Splitter is located between the bending part 6 and the flexible part 7 of the insertion tubes 2 what is shown in Fig. 11 and Fig 10. Preferably, the angle between the axes of the channels 38 is 90 degrees. Separate tension cable channels are led through the bending part 6 of the insertion tube to the distal tip 8. In this embodiment, splitter directs the cables from air channel 33 as shown in Fig. 8 and Fig. 10 to separate cable channels 38. Air is supplied through the flexible part 6 of the insertion tube via cable channels 38.

Mounting and removing the optical-illumination module into and from the endoscope.

The assembly and disassembly of the optical-illumination module 1 into and from the endoscope is carried out similarly as in the first example with an exception of unlocking of the capsule 5 from the distal tip 8.

After inserting the capsule 5 into the distal tip 8, the fold 27 is inserted into the locking groove 39 in the optical-illumination channel of the distal tip,as shown in Fig. 11. The endoscope is now ready to use. After completing the medical procedure for uninstallation, the optical-illumination module should be slightly pushed out of the insertion tube to release the fold 27 from the locking groove 39 of the capsule 5. the remaining steps are analogous to example 1.

### Building and function - example 3.

In this embodiment, the function and design of the optical-illumination module 1 and the insertion tube 2 is analogous to example 1 with the exceptions:
In this embodiment, the tension cables are guided through the optical-illumination channel 31 starting from the handle 9 up to the distal tip 8 due to the clearance between the optical-illumination cable 4 and the optical-illumination channel 31 created by diameter differences, shown in Fig. 7.

The fold of on a capsule 5 has the form of an elastic plate - spring fold 28 - made of metal or plastic with selected stiffness in the shape of a wave protruding beyond the outer surface of the capsule 5. The spring fold 28 is mounted in the socket for spring fold 42. The socket for spring fold 42 in the capsule housing is shape adapted to the spring fold 28 so that the spring fold could fit in it when under the pressure but could not fall out of the socket. When the capsule is inserted into the optical-illumination channel at the distal end 8, the spring fold under pressure of the wall of the insertion tube channel retracts into the socket until it hits the locking groove 39 where, without pressure from the channel wall, it returns to its previous shape. The spring fold 28 partially enters the locking groove 39 to keep the capsule and the entire optical-illumination module at the same time stable in the insertion tube.

### Mounting and removing the optical-illumination module into and from the endoscope

Assembly and disassembly of the optical-illumination module from the endoscope is carried out in the same way described in Example 2. The spring fold 28 under the pressure of the wall of the optical-illumination channel during insertion or retraction of the optical-illumination module 1 is deformed, allowing for assembling or disassembling the module.

### Building and function - example 4.

In this embodiment, the function and design of the optical-illumination module 1 is analogous to example 1 with the exceptions:
The tension cables directed in the handle 9 are guided through a dedicated tension cable channel 37 Fig. 9. Due to the narrow tension cable channel, in this embodiment splitter 41 oftension cable channel is applied. Splitter is a part of the insertion tube made of metal such as steel or polymer e.g. polyethylene or a flexible material such as silicone or rubber. Splitter is designed to provide the continuation of the insertion tube's functional channels and to separate the channel where the tension cables are guided into four separate tension cable channels 38 close to the insertion tube perimeter as shown in Fig. 10. Splitter is positioned between the bending part 6 and the flexible part 7 as shown in Fig. 11. Preferably, the angle between the axes of the channels 38 is 90 degrees. Splitter has the continuation of each channel through the insertion tube 2, and proximally a single input channel is routed in the flexible part 7 of the insertion tube, and the distally four channels near the perimeter of the bending part 6 of the insertion tube. Separate tension cable channels lead through the entire bending part 6 to the distal tip 8.

Mounting and removing the optical-illumination module into and from the endoscope is analogous to example 1.

### References to the drawing:

1 - Optical-illumination module
2 - Insertion tube
3 - Electro-optical connector
4 - Optical-illumination cable
5 - Capsule
6 - Bending part
7 - Flexible part
8 - Distal tip
9 - Handle
10 - Optical channel output
11 - Tool channel input
12 - Plug for closing tool channel
13 - Air channel inlet
14 - Water channel inlet
15 - Suction channel outlet
16 - Knobs
17 - Lens
18 - Electrical wires bundle
19 - Optical fibers bundle
20 - Stiffening rod
21 - Spiral protective band
22 - Elastic sheath
23 - Electrical conductors
24 - Insulator
25 - Transparent window
26 - Capsule back wall
27 - Fold
28 - Spring fold
29 - Bending section
30 - Flexible section
31 - Optical-illumination channel
32 - Tension cables
33 - Air channel
34 - Tool channel
35 - Suction channel
36 - Water channel
37 - Tension cable channel
38 - Tension cable channels of bending part
39 - Locking groove
40 - Blocking flange
41 - Splitter
42 - Socket for spring fold

## Claims

1. An endoscope comprising a handle (9) connected to a flexible insertion tube (2) in the form of a flexible cable with a bundle of functional channels and tension cables for moving the tip of the insertion tube,
said endoscope further comprising a removable and independent optical illumination module 1,
wherein said insertion tube includes an air channel (33), a suction channel (35), a water channel (36) and an optical illumination channel (31), and
wherein said insertion tube includes a flexible part (7) that leads from the handle, a bending part (6) located distal to the flexible part (7) and a distal tip (8),
wherein said optical illumination module (1) comprises a camera enclosed in a capsule (5) having a diameter, an optical fiber bundle (19) and an electric wires bundle (18),
wherein said bundles (19, 18) are guided in said optical illumination module (1) within an optical illumination cable (4) which extends from the proximal side of the capsule (5),
wherein an electro-optical connector (3) which is a connector for peripheral devices is located at the proximal-most end of said cable (4) and terminates said cable (4) and in which the optical fiber bundle (19) and the electric wires bundle (18) terminate,
said optical illumination module (1) being configured to be guided through the entire length of the insertion tube within the optical illumination channel (31),
wherein along the length of the distal tip (8) of the insertion tube (2) the optical illumination channel (31) has a diameter equal to or greater than the diameter of the capsule (5) of the optical illumination module (1) and wherein the diameter of the optical illumination channel (31) in the bending part (6) and the flexible part (7) of the insertion tube (2) is smaller than the diameter of the capsule (5) of the independent optical illumination module (1),
wherein the narrowing of diameter of the optical illumination channel (31) between the distal part (2) of the insertion tube and the bending part (6) of the insertion tube is such that it forms a blocking flange (40) for the capsule (5) such that when the optical illumination module (1) is installed within the optical illumination channel (31) the capsule (5) is blocked by the blocking flange (40) such that the optical illumination module (1) comprising the capsule (5) is mounted in the distal tip (8) of the endoscope,
wherein the optical illumination cable (4) of the optical illumination module (1) which extends from the proximal side of capsule (5) is of a length such that when said cable (4) is guided through the bending part (6) and the flexible part (7) of the insertion tube (2) the electro-optical connector (3) thereof is led out of an optical channel output (10) located in the handle (9) of the endoscope and which communicates with the optical illumination channel (31) of the insertion tube (2) such that said connector (3) extends outside said output (10),
wherein the optical illumination cable (4) has a bending section (29) and a flexible section (30) and is smaller in diameter than the optical illumination channel (31) but adapted to the size of the optical-illumination channel in the flexible part (7) and the bending part (6),
said cable (4) further comprises a stiffening rod (20) which extends in a longitudinal direction along the length of said cable (4), wherein the optical fiber bundle (19) and the electrical wires bundle (18) are located around said rod (20) and wherein a hermetic elastic sheath (22) forms the outer layer of said cable (4),
wherein the lengths of the bending section (29) and the flexible section (28) of the optical illumination cable (4) correspond to the lengths of the bending part (6) and the flexible part (7) of the insertion tube (2) respectively.

2. Endoscope according to claim 1, **characterized in that** the tension cables are routed in the optical-illumination channel (31).

3. Endoscope according to claim 1, **characterized in that** the tension cables are routed in the air channel (33).

4. Endoscope according to claim 1, **characterized in that** the tension cables are routed in the tension cable channel (37)

5. Endoscope according to claim 1, wherein the stiffening rod (20) has the same stiffness over its entire length.

6. Endoscope according to claim 1, **characterized in that** the optical-illumination cable (4) additionally comprises a spiral protective band (21) attached to the rear wall of the capsule (5) and to the electro-optical connector (3).

7. The endoscope according to claim 3 and claim 4, **characterized in that** between the flexible part (7) and the bending part (6) of the insertion tube (2) a channel splitter (41) is applied.

8. The endoscope according to claim 1, **characterized in that** the electro-optical connector (3) has a smaller diameter than the optical-illumination channel (31) along its entire length.

9. The endoscope according to claim 1, **characterized in that** the electro-optical connector (3) has a cylindrical shape.

10. The endoscope according to claim 1, **characterized in that** the electro-optical connector (3) has electric conductors (23) separated by electric insulators (24) located on the side wall of the electro-optical connector (3).

11. The endoscope according to claim 1, wherein the electro-optical connector (3) is equipped with a transparent window (25).

12. Endoscope according to claim 1, **characterized in that** the capsule (5) is equipped with a spring fold (28) matching in shape to a corresponding locking groove (39) of the distal tip (8) so as to fix the capsule (5) in the distal end (8).

13. Endoscope according to claim 1, **characterized in that** the capsule (5) is equipped with a fold (27) matching in size and shape to the locking groove (39), blocking the capsules (5) in the distal tip (8).

## Patentansprüche

1. Endoskop mit einem Griff (9), der mit einer flexiblen Spekulumsonde (2) in Form eines flexiblen Kabels mit einem Bündel funktioneller Kanäle und Fäden zurm Bewegung der Sondenspitze verbunden ist,
das genannte Endoskop besitzt ein abnehmbares und unabhängiges Optik- und Beleuchtungsmodul (1),
in welchem die Sichtglassonde einen Luftdurchgang (33), Saugdurchgang (35), Wasserdurchgang (36) und optischen Beleuchtungsdurchgang (31) umfasst, und
in welchem die Spekulumsonde einen flexiblen und von dem Griff wegführenden Teil (7), einen flexiblen Teil (6), der an der distalen Seite des elastischen Teils (7) angeordnet ist, eine distale Spitze (8) umfasst,
in welchem das optische Beleuchtungsmodul (1) eine in einer Kapsel (5) mit einem Durchmesser eingeschlossene Kamera, ein Bündel optischer Fasern (19), ein elektrisches Bündel (18) umfasst,
in welchem die Bündel (19, 18) in dem optischen Beleuchtungsmodul (1) innerhalb eines optischen Beleuchtungskabels (4) geführt sind, das sich von der proximalen Seite der Kapsel (5) erstreckt,
in welchem der elektrooptische Verbinder (3) ein peripherer Verbinder ist, der am proximalen Kabelende (4) angeordnet ist und das Kabel (4) abschließt, und in welchem das optische Faserbündel (19) und das elektrische Bündel (18) enden,
in welchem das optische Beleuchtungsmodul (1) konfiguriert ist, um die gesamte Länge der Schauglassonde in dem optischen Beleuchtungskanal (31) zu führen,
in welchem der optische Beleuchtungskanal (31) über die gesamte Länge der distalen Spitze (8) der Sonde (2) einen Durchmesser aufweist, der gleich oder größer als der Durchmesser der Kapsel (5) des optischen Beleuchtungsmoduls (1) ist und außerdem ist der Durchmesser des optischen Beleuchtungskanals (31) im flexiblen Teil (6) und im elastischen Teil (7) der Schauglassonde (2) kleiner als der Durchmesser der Kapsel (5) des unabhängigen optischen Beleuchtungsmoduls (1),
in welchem die Verengung des Durchmessers des optischen Beleuchtungskanals (31) zwischen dem distalen Teil (2) der Spekulumsonde und dem flexiblen Teil (6) der Spekulumsonde derart ist, dass sie einen Verriegelungskragen (40) der Kapsel (5) bildet, so dass, wenn das optische Beleuchtungsmodul (1) innerhalb des optischen Beleuchtungskanals (31) installiert ist, die Kapsel (5) durch diesen Verriegelungskragen (40) verriegelt wird, so dass das optische Beleuchtungsmodul (1) mit der Kapsel (5) im distalen Ende (8) des Endoskops montiert ist,
in welchem das optische Beleuchtungskabel (4) des optischen Beleuchtungsmoduls (1), das sich von der proximalen Seite der Kapsel (5) erstreckt, eine solche Länge hat, dass, wenn das Kabel (4) durch den flexiblen Teil (6) geführt wird, und am flexiblen Teil (7) der Spekulumsonde (2) der elektrisch-optische Stecker (3) aus der im Endoskopgriff (9) befindlichen Öffnung des optischen Kanals (10) herausgeführt wird und welcher sich mit dem optischen Beleuchtungskanal (31) der Spekulumsonde (2) auf die Art und Weise kommuniziert, dass sich das Gelenk (3) über den Mund (10) hinaus erstreckt,
in welchem das Lichtleiterkabel (4) einen flexiblen Abschnitt (29) und einen elastischen Abschnitt (30) und einen kleineren Durchmesser als der Lichtleiterkanal (31) aufweist, jedoch an die Abmessung des Lichtleiterkanals im elastischen Teil (7) und flexiblen Teil (6) angepasst ist,
das obige Kabel (4) hat einen Versteifungskern (20), der sich in Längsrichtung entlang der gesamten Kabellänge (4) erstreckt, in dem ein Bündel optischer Fasern (19) und ein elektrisches Bündel (18) um den Kern (20) angeordnet sind und wo ein hermetisch flexibler Mantel (22) die äußere Kabeloberfläche (4) bildet,
wobei die Länge des flexiblen Abschnitts (29) und des elastischen Abschnitts (30) des Lichtleiterkabels (4) jeweils der Länge des flexiblen Teils (6) und des elastischen Teils (7) entsprechen.

2. Endoskop nach Anspruch 1, wobei, dass die Fäden durch einen optischen Beleuchtungskanal (31) geführt sind.

3. Endoskop nach Anspruch 1, wobei, dass die Fäden durch einen optischen Luftkanal (33) geführt sind.

4. Endoskop nach Anspruch 1, wobei, dass die Fäden durch einen optischen Beleuchtungskanal (37) geführt sind.

5. Endoskop nach Anspruch 1, wobei, dass der Versteifungskern (20) über seine gesamte Länge die gleiche Steifigkeit aufweist

6. Endoskop nach Anspruch 1, wobei, dass das optische Beleuchtungskabel (4) zusätzlich mit einem spiralförmigen Schutzband (21) versehen ist, das an der Rückwand der Kapsel (5) und am elektrisch-optischen Anschluss (3) angebracht ist.

7. Endoskop nach Anspruch 3 und 4, wobei, dass zwischen dem elastischen Teil (7) und dem flexiblen Teil (6) der Sonde (2) ein Kanalteiler (41) vorhanden ist.

8. Endoskop nach Anspruch 1, wobei, dass der elektrooptische Anschluß (3) über seine gesamte Länge einen kleineren Durchmesser als der optische Beleuchtungskanal (31) hat

9. Endoskop nach Vorbehalt 1, **gekennzeichnet dadurch, dass** der elektrooptische Anschluss (3) eine zylindrische Form hat

10. Endoskop nach Vorbehalt 1, **gekennzeichnet dadurch, dass** der elektrooptische Verbinder (3) elektrische Leiter (23) aufweist, die durch elektrische Isolatoren (24) getrennt werden, die sich auf der Seite der Verbinderwand (3) befinden.

11. Endoskop nach Vorbehalt 1, **gekennzeichnet dadurch, dass** der elektrooptische Anschluss (3) mit einem transparenten Anschlussfenster (25) ausgestattet wird.

12. Endoskop nach Vorbehalt 1, **gekennzeichnet dadurch, dass** die Kapsel (5) mit einem federbelasteten Vorsprung (28) versehen wird, der passend zu der entsprechenden Verriegelungsnut (39) am distalen Ende (8) geformt ist, um die Kapsel ( 5) im distalen Ende (8) zu montieren.

13. Endoskop nach Vorbehalt 1, **gekennzeichnet dadurch, dass** die Kapsel (5) mit einem der entsprechenden Rastnut (39) formangepaßten Vorsprung (27) ausgestattet ist, welcher die Kapsel (5) im distalen Ende (8) fixiert.

## Revendications

1. Endoscope comprenant une poignée (9) reliée à une sonde souple (2) sous la forme d'un tube souple avec un faisceau de canaux fonctionnels et des cordons pour déplacer la pointe de la sonde,
ledit endoscope comprend un module optique et d'éclairage amovible et indépendant (1),
dans lequel ladite sonde comprend un canal d'air (33), un canal d'aspiration (35), un canal d'eau (36) et un canal optique et d'éclairage (31), et
dans lequel ladite sonde comprend une partie élastique (7), allant de la poignée, une partie flexible (6) située du côté distal de la partie élastique (7), une pointe distale (8),
dans lequel ledit module optique et d'éclairage (1) comprend une caméra enfermée dans une capsule (5) ayant un diamètre, un faisceau de fibres optiques (19), un faisceau électrique (18),
dans lequel ledits faisceaux (19, 18) sont guidés dans ledit module optique et d'éclairage (1) à l'intérieur du câble optique et de d'éclairage (4), qui s'étend depuis le côté proximal de la capsule (5),
dans lequel le connecteur électrique et optique (3) est un connecteur pour les dispositifs périfériques et est situé à l'extrémité proximale dudit câble (4) et termine ledit câble (4), et dans lequel le faisceau de fibres optiques (19) et le faisceau électrique (18) se terminent,
dans lequel ledit module optique et d'éclairage (1) est configuré pour guider sur toute la longueur la sonde dans le canal optique et d'éclairage (31),
dans lequel, sur toute la longueur de la pointe distale (8) de la sonde (2), le canal optique et d'éclairage (31) a un diamètre égal ou supérieur au diamètre de la capsule (5) du module optique et d'éclairage (1) et en outre le diamètre du canal optique et d'éclairage (31) dans la partie flexible (6) et dans la partie élastique (7) de la sonde (2) est inférieur au diamètre de la capsule (5) du module optique et d'éclairage indépendant (1),
dans lequel le rétrécissement du diamètre du canal optique et d'éclairage (31) entre la partie distale (2) de la sonde et la partie flexible (6) de la sonde est tel qu'il forme un collier de blocage (40) de la capsule (5) de sorte que lorsque le module optique et d'éclairage (1) est installé à l'intérieur du canal optique et d'éclairage (31), la capsule (5) est bloquée par ledit collier de blocage (40) de sorte que le module optique et d'éclairage (1) contenant la capsule (5) est monté dans la pointe distale (8) de l'endoscope,
dans lequel le câble optique et d'éclairage (4) du module optique et d'éclairage (1), qui s'étend du côté proximal de la capsule (5) est d'une telle longueur que ledit câble (4) est guidé par la partie flexible (6) et la partie élastique (7) de la sonde (2), le connecteur électrique et optique (3) sort de l'extrémité du canal optique (10) situé dans la poignée (9) de l'endoscope et qui communique au canal optique et d'éclairage (31) de la sonde (2) de sorte que ledit connecteur (3) dépasse la sortie (10),
dans lequel le câble optique et d'éclairage (4) a une section flexible (29) et une section élastique (30) et son diamètre est inférieur au diamètre du canal otpique et d'éclairage (31) mais ajusté à la taille du canal otpique et d'éclairage dans la partie élastique (7) et la partie flexible (6),
ledit câble (4) comprend une âme raidissante (20) qui s'étend longitudinalement sur toute la longueur dudit câble (4), dans laquelle le faisceau de fibres optiques (19) et le faisceau électrique (18) sont disposés autour de l'âme (20) et où la gaine flexible hermétique (22) forme la surface extérieure du câble (4),
et la longueur de la section flexible (29) et de la section élastique (30) du câble optique et d'éclairage (4) correspond respectivement à la longueur de la partie flexible (6) et de la partie (7).

2. Endoscope selon la revendication 1, **caractérisé en ce que** les cordons sont guidés à travers le canal optique et d'éclairage (31)

3. Endoscope selon la revendication 1, **caractérisé en ce que** les cordons sont guidés à travers le canal d'air (33)

4. Endoscope selon la revendication 1, **caractérisé en ce que** les cordons sont guidés à travers le canal de cordons (37)

5. Endoscope selon la revendication 1, **caractérisé en ce que** l'âme raidissante (20) a la même rigidité sur toute la longueur

6. Endoscope selon la revendication 1, **caractérisé en ce que** câble optique et d'éclairage (4), est équipé en plus d'une bande de protection en spirale (21), fixée à la paroi arrière de la capsule (5) et au connecteur électrique et optique (3)

7. Endoscope selon les revendications 3 et 4, **caractérisé en ce que** parmi la partie élastique (7) et la partie flexible (6) de la sonde (2) est situé un séparateur de canaux (41)

8. Endoscope selon la revendication 1, **caractérisé en ce que** le connecteur électrique et optique (3) a un diamètre inférieur au diamètre du canal optique et d'éclairage (31) sur toute sa longueur

9. Endoscope selon la revendication 1, **caractérisé en ce que** le connecteur électrique et optique (3) a une forme cylindrique

10. Endoscope selon la revendication 1, **caractérisé en ce que** le connecteur électrique et optique (3) a des conducteurs électriques (23) séparés par des isolants électriques (24) situés sur le côté latéral de la paroi du connecteur (3)

11. Endoscope selon la revendication 1, **caractérisé en ce que** le connecteur électrique et optique (3) est équipé d'une fenêtre de connexion transparente (25)

12. Endoscope selon la revendication 1, **caractérisé en ce que** la capsule (5) est équipée d'une languette à ressort (28) dont la forme est ajustée à la rainure de blocage correspondante (39) sur l'extrémité distale (8) de sorte pour fixer la capsule (5) dans l'extrémité distale (8).

13. Endoscope selon la revendication 1, **caractérisé en ce que** la capsule (5) est équipée d'une languette (27) dont la forme est ajustée à la rainure de blocage correspondante (39), fixant la capsule (5) dans l'extrémité distale (8).
